# EUROPÄISCHE PATENTSCHRIFT

(11) **EP 2 890 422 B1**
(45) Veröffentlichungstag und Bekanntmachung des Hinweises auf die Patenterteilung: **05.10.2016**
(21) Anmeldenummer: 13753582.9
(22) Anmeldetag: 19.08.2013
(51) Int. Cl.: A61M 1/36, A61B 5/02, A61F 13/42, G01M 3/16

(54) **VERFAHREN UND VORRICHTUNG ZUM PRÜFEN VON AUF DIE HAUT EINES PATIENTEN AUFZUBRINGENDEN SENSOREN ZUM ERKENNEN VON FLÜSSIGKEIT ODER FEUCHTIGKEIT**
METHOD AND DEVICE FOR EXAMINING SENSORS TO BE APPLIED TO THE SKIN OF A PATIENT FOR DETECTING FLUIDS OR MOISTURE
PROCÉDÉ ET DISPOSITIF DE CONTRÔLE DE CAPTEURS DEVANT ÊTRE APPLIQUÉS SUR LA PEAU D'UN PATIENT POUR DÉTECTER LA PRÉSENCE DE LIQUIDE OU D'HUMIDITÉ

(30) Priorität: 31.08.2012 DE 102012017205; 31.08.2012 US 201261695358 P
(43) Veröffentlichungstag der Anmeldung: 08.07.2015
(73) Patentinhaber: Fresenius Medical Care Deutschland GmbH, 61352 Bad Homburg (DE)
(72) Erfinder: SCHRÖRS, Alexander, 60389 Frankfurt (DE); HEPPE, John, 66606 St.Wendel (DE); RULLOF, Roland, 66822 Lebach (DE)
(74) Vertreter: Oppermann, Frank
(86) Internationale Anmeldenummer: PCT/EP2013/002486
(87) Internationale Veröffentlichungsnummer: WO 2014/032778

(56) Entgegenhaltungen:
- WO-A1-2006/008866
- WO-A1-2010/091852
- DE-A1-102010 012 545
- US-A1- 2002 198 483

## Beschreibung

Die Erfindung betrifft ein Verfahren und eine Vorrichtung zum Prüfen von auf die Haut eines Patienten aufzubringenden Sensoren zum Erkennen von Flüssigkeit oder Feuchtigkeit, insbesondere Feuchtigkeitssensoren zur Überwachung des Gefäßzugangs bei einer extrakorporalen Blutbehandlung, bei der Blut eines Patienten über eine arterielle Schlauchleitung, die eine arterielle Kanüle aufweist, von dem Patienten abgeführt wird, und über eine venöse Schlauchleitung, die eine venöse Punktionskanüle aufweist, dem Patienten zugeführt wird. Darüber hinaus betrifft die Erfindung ein Verfahren zum Produzieren von auf die Haut eines Patienten aufzubringenden Sensoren zum Erkennen von Flüssigkeit oder Feuchtigkeit.

Aus der WO 2011/116943 ist ein Feuchtigkeitssensor zur Überwachung eines Gefäßzugangs bekannt, der als ein textiles Flächengebilde aus nicht-leitfähigen Kettfäden und nicht-leitfähigen Schussfäden sowie leitfähigen Kettfäden und leitfähigen Schussfäden ausgebildet ist. Die nicht-leitfähigen Kett- und Schussfäden sowie leitfähigen Kett- und Schussfäden sind in dem Gewebe derart angeordnet, dass sich eine elektrisch leitende Struktur mit elektrischen Anschlüssen ergibt. Zur Erkennung von Flüssigkeit oder Feuchtigkeit wird der elektrische Widerstand zwischen den Anschlüssen gemessen. Wenn das Gewebe mit Flüssigkeit benetzt wird, verändert sich der elektrische Widerstand, so dass die Flüssigkeit erkannt wird. Die WO 2011/116943 schlägt bei den Feuchtigkeitssensoren, deren Leiterbahnen durch eine Mehrzahl von leitfähigen Fäden gebildet werden, eine Überprüfung jeder einzelnen Leiterbahn im Herstellungsprozess auf ihre Funktionsfähigkeit vor.

Die WO 2010/091852 beschreibt einen textilen Feuchtigkeitssensor zur Überwachung eines Gefäßzugangs, bei dem die elektrisch leitende Struktur mit den elektrischen Anschlüssen auf das Gewebe aufgedruckt ist.

Neben den resistiven Sensoren sind auch kapazitive Feuchtigkeitssensoren bekannt, die über eine elektrisch leitende Struktur verfügen, die sich aus mehreren elektrisch leitenden Abschnitten zusammensetzt.

Die bekannten Feuchtigkeitssensoren zur Überwachung eines Gefäßzugangs, die über eine elektrisch leitende Struktur verfügen, die sich aus mehreren elektrisch leitenden Abschnitten zusammensetzt, erlauben eine Überprüfung der Funktionsfähigkeit dadurch, dass die elektrischen Eigenschaften des Sensors unter definierten Bedingungen gemessen und mit als Referenzwert vorgegebenen Eigenschaften verglichen werden. Wenn die Abweichungen zwischen den gemessenen Eigenschaften und den als Referenzwert vorgegebenen Eigenschaften ein vorbestimmtes Maß überschreiten oder unterschreiten, beispielsweise wegen eines Leiterbahnbruchs oder Kurzschlusses, wird auf einen fehlerhaften Feuchtigkeitssensor geschlossen.

Die bekannten textilen Feuchtigkeitssensoren werden an der Punktionsstelle auf die Haut des Patienten aufgebracht. Zur Überwachung eines Gefäßzugangs bei einer extrakorporalen Blutbehandlung werden die Feuchtigkeitssensoren beispielsweise auf den Unterarm des Patienten aufgeklebt. Der Unterarm mit Gefäßzugang wird von dem Patienten während der extrakorporalen Blutbehandlung im Allgemeinen ruhig gehalten. Wenn der Patient während der Blutbehandlung aber den Unterarm bewegt, ist der Feuchtigkeitssensor mechanischen Beanspruchungen ausgesetzt, so dass die leitenden Abschnitte der elektrisch leitenden Struktur wiederholt Zug- Druck- oder Biegebeanspruchungen ausgesetzt sind. Das Problem der mechanischen Beanspruchung stellt sich bei textilen Feuchtigkeitssensoren, bei denen Kett- und Schussfäden die elektrisch leitende Struktur bilden. Aber auch bei gedruckten Leiterbahnen, besteht die Gefahr des Leiterbahnbruchs durch Mikrorisse.

Die textilen Feuchtigkeitssensoren mit gewebten oder gedruckten Leiterbahnen haben den Vorteil, dass sich die Sensoren in großen Stückzahlen auf einer gemeinsamen Gewebebahn kostengünstig herstellen lassen. Nach dem Weben und gegebenenfalls weiteren Bearbeitungsschritten werden die einzelnen Feuchtigkeitssensoren voneinander separiert.

Der Erfindung liegt die Aufgabe zugrunde, ein Verfahren für die Produktion von Feuchtigkeitssensoren mit einer elektrisch leitenden Struktur aus mehreren elektrisch leitenden Abschnitten in großen Stückzahlen anzugeben, wobei die hergestellten Feuchtigkeitssensoren hohen Qualitätsanforderungen genügen.

Eine weitere Aufgabe der Erfindung liegt darin, ein Verfahren zur Qualitätskontrolle von Feuchtigkeitssensoren mit einer elektrisch leitenden Struktur aus mehreren elektrisch leitenden Abschnitten anzugeben.

Darüber hinaus ist eine Aufgabe der Erfindung, eine Vorrichtung zu schaffen, die eine zuverlässige Qualitätskontrolle der Feuchtigkeitssensoren erlaubt.

Die Lösung dieser Aufgaben erfolgt erfindungsgemäß mit den Merkmalen der unabhängigen Patentansprüche. Die abhängigen Ansprüche betreffen bevorzugte Ausführungsformen der Erfindung.

Das erfindungsgemäße Verfahren und die erfindungsgemäße Vorrichtung beruhen auf der zerstörenden Prüfung eines oder mehrerer Feuchtigkeitssensoren, die der laufenden Produktion als Probe entnommen werden. Die Erfindung sieht eine komplexe mechanische Beanspruchung der Feuchtigkeitssensoren vor, die einer Beanspruchung nachempfunden wird, die in der Praxis auftritt, wenn der Feuchtigkeitssensor auf die Haut des Patienten, insbesondere dessen Unterarm aufgebracht, insbesondere aufgeklebt wird. Dabei können die mechanischen Beanspruchungen simuliert werden, die im ungünstigsten Fall auftreten können. Für den Fall, dass der oder die Feuchtigkeitssensoren einer Charge bei der Prüfung den gesetzten Anforderungen nicht entsprechen sollten, wird auf eine Fehlerhaftigkeit der Charge geschlossen. Es können dann weitere Proben genommen oder die gesamte Charge verworfen werden.

Es hat sich in Versuchen gezeigt, dass die mechanischen Beanspruchungen des Feuchtigkeitssensors auf der Haut des Patienten dadurch simuliert werden können, wenn der Feuchtigkeitssensor auf einen verformbaren Körper aufgebracht wird, der mehrfach verformt wird. Dabei treten in dem Feuchtigkeitssensor wechselnde Zug-, Druck- und Biegespannungen auf, die zu entsprechenden reversiblen und irreversiblen Verformungen im mikroskopischen Bereich und sogar zum Bruch der Leiterbahnen führen können. Die Prüfung entspricht also einer künstlichen Beanspruchung unter realitätsnahen Bedingungen, wobei eine vorgegebene Anzahl von Lastwechseln erzeugt wird. Der Feuchtigkeitssensor kann auf die Oberfläche des elastischen Körpers geklebt werden. Es ist aber auch jede andere Art der Befestigung des Feuchtigkeitssensors möglich. Entscheidend ist, dass der Feuchtigkeitssensor mit seiner Unterseite fest mit der Oberseite des elastischen Körpers verbunden ist.

Weiterhin hat sich in Versuchen gezeigt, dass eine besonders realitätsnahe Simulation der beim Tragen des Feuchtigkeitssensors auftretenden mechanischen Beanspruchungen dann möglich ist, wenn der Feuchtigkeitssensor auf die Oberfläche eines elastischen Torsionskörpers aufgebracht und der Torsionskörper mehrfach tordiert wird, beispielsweise oszillierend tordiert wird. Besonders bevorzugt wird ein zylindrischer Torsionskörper, auf dessen Mantelfläche der Feuchtigkeitssensor aufgebracht wird.

Während der extrakorporalen Blutbehandlung, insbesondere Hämodialysebehandlung, hält der Patient seinen Arm im Allgemeinen derart, dass die Handfläche nach oben zeigt. Die Erfinder haben erkannt, dass in der Praxis eine maximale Beanspruchung des Feuchtigkeitssensors nur dann auftreten kann, wenn der Patient seinen Arm um 180° dreht, so dass die Handfläche nach unten zeigt. Mit der Torsion des zylindrischen Torsionskörpers um einen Winkel von 180° kann also der maximale Bewegungsspielraum des Arms realitätsnah nachempfunden werden.

Die erfindungsgemäße Prüfung sieht eine Torsion des Torsionskörpers um einen vorgegebenen Torsionswinkel vor, der vorzugsweise zwischen 60 bis 180°, insbesondere 80 bis 160°, vorzugsweise 100 bis 140° liegt. Darüber hinaus sieht die erfindungsgemäße Prüfung eine Anzahl von Torsionen vor, die zwischen 100 und 1000, insbesondere 200 bis 800, vorzugsweise 400 bis 600 Torsionen liegt. Dadurch wird erreicht, dass der Feuchtigkeitssensor relativ großen Lastwechseln ausgesetzt ist, die zu einer Zerstörung des Sensors führen können.

Der Torsionskörper wird vorzugsweise an einem Ende fest eingespannt, und wird an dem anderen Ende um einen vorgegebenen Drehwinkel verdreht. Es ist aber auch möglich, den Torsionskörper an beiden Enden in entgegengesetzte Drehwinkel zu verdrehen.

Das erfindungsgemäße Verfahren und die erfindungsgemäße Vorrichtung bieten insbesondere bei der Prüfung von Feuchtigkeitssensoren besondere Vorteile, die als ein textiles Flächengebilde aus nicht leitfähigen Kettfäden und nicht leitfähigen Schussfäden sowie leitfähigen Kettfäden und leitfähigen Schussfäden ausgebildet sind, wobei die nicht leitfähigen Kett- und Schussfäden sowie leitfähigen Kett- und Schussfäden derart angeordnet sind, dass die elektrisch leitende Struktur ausgebildet ist. Das erfindungsgemäße Verfahren und die erfindungsgemäße Vorrichtung können aber auch zur Qualitätskontrolle von Feuchtigkeitssensoren eingesetzt werden, die ein Trägermaterial aufweisen, auf das die elektrisch leitende Struktur aufgebracht, vorzugsweise aufgedruckt ist. Grundsätzlich sind das erfindungsgemäße Verfahren und die erfindungsgemäße Vorrichtung zum Prüfen von allen Arten von Pflastersensoren und auch von Arzneimittel spendenden Pflastern geeignet.

Für die Erfindung ist grundsätzlich unerheblich, welche elektrischen Eigenschaften zur Qualitätskontrolle mit einem Referenzwert verglichen werden. Bei resistiven Feuchtigkeitssensoren beispielsweise wird der elektrische Widerstand und bei kapazitiven Feuchtigkeitssensoren die Kapazität gemessen. Die elektrischen Eigenschaften können vor und/oder nach der Wechselbeanspruchung und/oder während der mechanischen Wechselbeanspruchung gemessen werden. Zur Festlegung des Referenzwertes können die charakteristischen Eigenschaften auch vor der Wechselbeanspruchung gemessen werden. Beispielsweise ist es möglich, die vor und nach der Wechselbeanspruchung ermittelten Messwerte miteinander zu vergleichen und einer statistischen Auswertung zu unterziehen.

In Versuchen hat sich gezeigt, dass sich die auf die Oberfläche des elastischen Körpers aufgebrachten, insbesondere aufgeklebten, Feuchtigkeitssensoren nicht oder nur schwer wieder entfernen lassen. Daher sieht die erfindungsgemäße Vorrichtung zur Durchführung des erfindungsgemäßen Verfahrens eine Betätigungseinheit zur auswechselbaren Aufnahme des elastischen Körpers vor, die derart ausgebildet ist, dass der elastische Körper verformt wird. Nach der Prüfung des Feuchtigkeitssensors kann der elastische Körper mit dem Feuchtigkeitssensor einfach verworfen und ein neuer elastischer Körper, auf den der Feuchtigkeitssensor aufgebracht wird, wieder in die Betätigungseinheit eingesetzt werden.

Die erfindungsgemäße Vorrichtung verfügt über eine Messeinheit zum Messen der elektrischen Eigenschaften der Feuchtigkeitssensoren und eine Auswerteinheit zum Auswerten der Messergebnisse der Messeinheit.

Eine besonders bevorzugte Ausführungsform sieht als Torsionskörper einen Schlauch vor. Dieser elastische Körper kommt dem Arm des Patienten am nächsten. Insbesondere werden durch den Schlauch Dehnungen und Stauchungen der Haut des Patienten besonders realitätsnah simuliert. Dabei können die mechanischen Beanspruchungen durch die Materialstärke und/oder die Materialeigenschaften des Schlauchs gezielt verändert werden. Es ist in einer weiteren Ausführungsform grundsätzlich auch möglich, einen Schlauch zu verwenden, der eine nur verhältnismäßig geringe Materialstärke hat. Die für die Prüfung des Feuchtigkeitssensors erforderlichen mechanischen Eigenschaften können in diesem Fall dadurch hergestellt werden, dass der Schlauch mit Druckkluft aufgeblasen wird. Hierzu kann eine Drucklufteinheit vorgesehen sein, die dem an beiden Enden luftdicht verschlossenen Schlauch über eine Druckluftzuführung Druckluft zuführt. Die Druckluft-Einheit kann an dem drehbaren und/oder feststehenden Körper vorgesehen sein, an denen die Schlauchenden luftdicht befestigt werden können, wobei die Druckluft über einen Kanal in dem drehbaren und/oder feststehenden Körper zugeführt werden kann.

Bei der bevorzugten Ausführungsform, die einen Schlauch als Torsionskörper vorsieht, weist die Betätigungseinheit vorzugsweise einen drehbaren zylindrischen Körper auf, an dem das eine Ende des Schlauchs befestigt werden kann und einen feststehenden zylindrischen Körper auf, an dem das andere Ende des Schlauchs befestigt werden kann. Es ist aber auch möglich, dass beide zylindrischen Körper der Betätigungseinheit gegeneinander verdrehbar sind.

Bei einer weiteren besonders bevorzugten Ausführungsform ist die Steuereinheit derart ausgebildet, dass die Betätigungseinheit eine vorgegebene Anzahl von Torsionen des Torsionskörpers vornimmt. Vorzugsweise ist die Steuereinheit derart ausgebildet, dass die Betätigungseinheit die Torsionen um einen vorgegebenen Torsionswinkel vornimmt. Die Anzahl der Torsionen und der Torsionswinkel können auf einer Eingabeeinheit eingegeben oder in der Steuereinheit fest vorgegeben sein. Die Steuereinheit kann eine automatische Steuerung des gesamten Prüfablaufs vorsehen.

Im Folgenden wird ein Ausführungsbeispiel der Erfindung unter Bezugnahme auf die Zeichnungen näher erläutert.

Es zeigen:
- Fig. 1: ein Ausführungsbeispiel eines textilen Feuchtigkeitssensors in vereinfachter schematischer Darstellung,
- Fig. 2: eine vereinfachte schematische Darstellung eines Ausführungsbeispiels der Vorrichtung zur Durchführung einer Prüfung von Feuchtigkeitssensoren,
- Fig. 3: einen Schnitt durch die Vorrichtung entlang der Linie I - I von Fig. 1 und
- Fig. 4: eine stark vereinfachte schematische Darstellung der Verfahrensschritte zur Herstellung des Feuchtigkeitssensors.

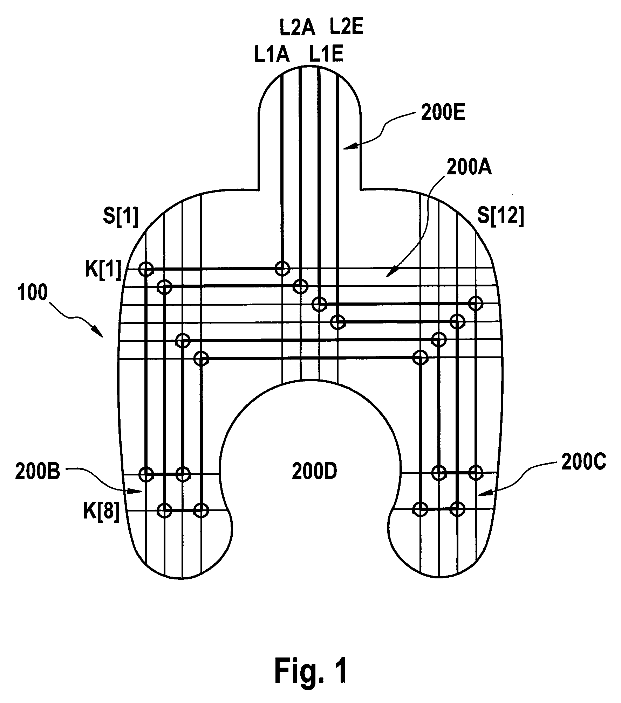

Fig. 1 zeigt ein Ausführungsbeispiel eines gewebten Feuchtigkeitssensors 100 zur Überwachung eines Gefäßzugangs in der Draufsicht. Der Feuchtigkeitssensor, der wie ein Pflaster gehandhabt werden kann, wird auf die Haut des Patienten aufgeklebt. Für die Überwachung eines arteriellen oder venösen Gefäßzugangs bei der extrakorporalen Blutbehandlung wird der Feuchtigkeitssensor auf den Unterarm des Patienten aufgeklebt.

Der Feuchtigkeitssensor 100 ist als ein auf die Haut des Patienten aufzulegendes Pad aus einem textilen Flächengebilde ausgebildet. Das textile Flächengebilde ist ein Gewebe, das aus elektrisch leitfähigen und elektrisch nicht leitfähigen Kett- und Schussfäden besteht. An den Kreuzungspunkten sind die elektrisch leitfähigen Kett- und Schussfäden derart angeordnet, dass eine elektrisch leitfähige Struktur gebildet wird.

Der Feuchtigkeitssensor weist einen mittleren Bereich 200A mit zwei Schenkeln 200B, 200C auf, die einen halbkreisförmigen Ausschnitt 200D seitlich umschließen. An den mittleren Bereich ist eine Lasche 200E angeformt, die beiden Schenkeln gegenüberliegt. Die elektrisch leitfähigen Kett- und Schussfäden, die eine Struktur aus elektrischen Leiterbahnen bilden, werden durch horizontale und vertikale dünne Linien gekennzeichnet. Die Schussfäden S verlaufen in vertikaler und die Kettfäden K in horizontaler Richtung. Die Leiterbahnstruktur wird von acht Kettfäden K [1] bis K [8] und zwölf Schussfäden S [1] bis S [12] gebildet, die an den Kreuzungspunkten derart angeordnet sind, dass sie entweder elektrisch leitend verbunden sind oder elektrisch voneinander isoliert sind.

In Fig. 1 sind die elektrischen Kontaktstellen an den Kreuzungspunkten zwischen den elektrisch leitfähigen Kett- und Schussfäden K [i], S [i] als Kreise dargestellt. Die erste Leiterbahn L1A-L1E verläuft von der Lasche 200E über den mittleren Bereich 200A zu dem linken Schenkel 200B und von dem linken Schenkel über den mittleren Bereich zu dem rechten Schenkel 200C und von dem rechten Schenkel über den mittleren Bereich zurück zu der Lasche des Pads. Der Anfang der jeweiligen Leiterbahn ist mit "A" und das Ende der Leiterbahn ist mit "E" bezeichnet. Die beiden Enden L1A, L1E der ersten Leiterbahn L1A-L1E bilden ein erstes Paar von Anschlüssen. Die zweite Leiterbahn L2A-L2E verläuft von der Lasche 200E über den mittleren Bereich 200A zu dem linken Schenkel 200B und von dem linken Schenkel über den mittleren Bereich zu dem rechten Schenkel 200C und von dem rechten Schenkel über den mittleren Bereich zu der Lasche des Pad 40. Die beiden Enden L2A, L2E der zweiten Leiterbahn L2A-L2E bilden ein zweites Paar von Anschlüssen. Zwischen den Anschlüssen L1A und L2E wird der elektrische Widerstand gemessen, während die Anschlüsse L1E und L2A mit einem nicht dargestellten elektrischen Abschlusswiderstand verbunden werden.

Der unter Bezugnähme auf Fig. 1 beschriebene Feuchtigkeitssensor ist im Einzelnen in der WO 2011/116943 beschrieben.

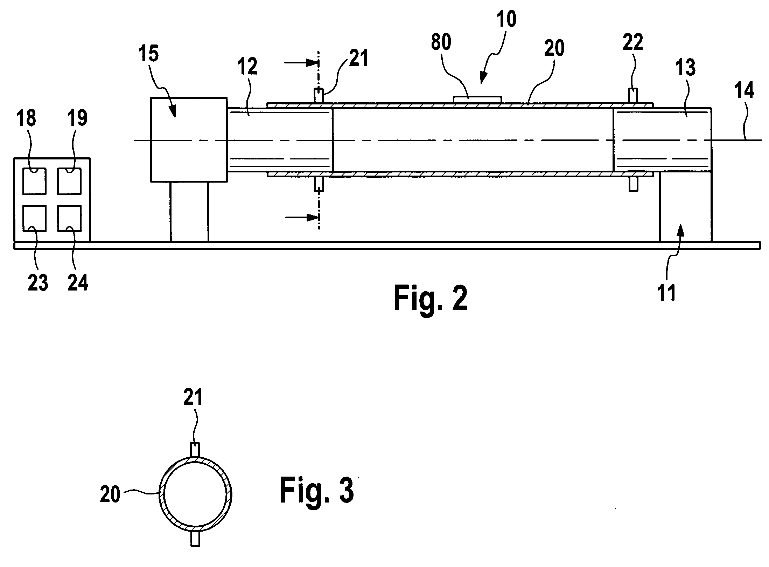

Fig. 2 zeigt in vereinfachter schematischer Darstellung ein Ausführungsbeispiel einer Vorrichtung 10 zum Prüfen von Feuchtigkeitssensoren, bei denen es sich insbesondere um textile Feuchtigkeitssensoren mit gewebten oder aufgedruckten Leiterbahnen handeln kann. Ein besonders bevorzugter Verwendungszweck liegt in der Prüfung von gewebten Feuchtigkeitssensoren der unter Bezugnahme auf Fig. 1 beschriebenen Art, die auf den Unterarm des Patienten aufgeklebt werden.

Die Vorrichtung 10 weist ein Gestell 11 auf, an dem zwei zylindrische Körper 12, 13, beispielsweise Dorne, auf einer gemeinsamen Achse 14 im Abstand zueinander angeordnet sind. Einer der beiden zylindrischen Körper 13 ist an dem Gestell 11 unbeweglich befestigt, während der andere zylindrische Körper 12 an dem Gestell um die Achse 14 drehbar angeordnet ist. In Fig. 2 ist der linke zylindrische Körper 12 um die Achse 14 drehbar gelagert und der rechte zylindrische Körper 13 fest mit dem Gestell 11 verbunden. Die Vorrichtung 10 verfügt über eine nur schematisch dargestellte Betätigungseinheit 15 für den drehbaren zylindrischen Körper 12. Die vorzugsweise elektromotorisch oder pneumatisch betriebene Betätigungseinheit 15 erlaubt eine Drehung des zylindrischen Körpers 12 im und entgegen des Uhrzeigersinns um einen vorgegebenen Drehwinkel.

Die Betätigungseinheit 15 wird von einer Steuereinheit 18 angesteuert. Die Steuereinheit 18 steuert die Betätigungseinheit 15 derart an, dass die Betätigungseinheit innerhalb eines Prüfzyklus eine vorgegebene Anzahl von Drehungen des zylindrischen Körpers 12 ausführt, wobei jeweils einer Drehung im Uhrzeigersinn um einen vorgegeben Winkel eine Drehung entgegen des Uhrzeigersinns um einen vorgegebenen Winkel folgt.

Die Anzahl der Drehungen und die Größe des Drehwinkels kann auf einer Eingabeeinheit 19 eingegeben werden. Der maximale Drehwinkel ist bei dem Ausführungsbeispiel auf 120° beschränkt. Für einen Prüfzyklus kann der zylindrische Körper 12 innerhalb eines vorgegeben Zeitintervalls, beispielsweise 2s, aus seiner Ausgangslage, beispielsweise um 120°, entgegen dem Uhrzeigersinn gedreht und dann im Uhrzeigersinn, beispielsweise um 120°, wieder in die Ausgangslage zurückgedreht werden, wobei die Drehung im und entgegen dem Uhrzeigersinn von der Steuereinheit 18 innerhalb des Prüfzyklus nach Ablauf eines vorgegebenen Zeitintervalls solange wiederholt wird, bis eine vorgegebene Anzahl von Drehungen, beispielsweise 500 Zyklen, beziehungsweise Lastwechsel, ausgeführt worden sind.

Die beiden zylindrischen Körper 12, 13 dienen zur Befestigung eines Schlauchs 20, dessen Innendurchmesser dem Außendurchmesser der zylindrischen Körper entspricht, so dass der Schlauch auf die zylindrischen Körper passend aufgeschoben werden kann. An beiden Enden wird der Schlauch mit in Fig. 2 nur andeutungsweise dargestellten Befestigungsmitteln 21, 22 befestigt, so dass die Schlauchenden die zylindrischen Körper luftdicht umschließen. Die Befestigungsmittel 21, 22 können im einfachsten Fall Kabelbinder sein. Für einen automatischen Betrieb der Vorrichtung sind aber selbsttätig arbeitende, beispielsweise pneumatisch oder elektromotorisch angetriebene Befestigungsmittel vorgesehen, die von der Steuereinheit angesteuert werden können.

Wenn die Schlauchenden an den zylindrischen Körpern 12, 13 befestigt sind, führt die Drehbewegung des linken zylindrischen Körpers zu einer Torsion des Schlauchs 20 um den vorgegebenen Torsionswinkel.

Mit dem mehrfach tordierten Schlauch 20 kann der Unterarm eines Patienten während der Dialysebehandlung nachgebildet werden. In dem vorliegenden Ausführungsbeispiel wird eine mittlere Behandlungszeit von 8 h angenommen. Der Schlauchdurchmesser sollte dem mittleren Durchmesser des Unterarms entsprechen.

Zur Ermittlung eines optimalen Auslenkwinkels wurde ein Gitter auf den Unterarm einer Versuchsperson aufgezeichnet, wobei der Unterarm mit der Handfläche nach oben weisend auflag, was einem Drehwinkel von 0° entspricht. Anschließend wurde das Gitter vermessen, als der Unterarm um 180° verdreht war, wobei die Handfläche nach unten wies. Es zeigte sich eine Verformung der orthogonal zueinander verlaufenden Linien.

Diese Verformung der Gitterlinien wurde durch eine Torsion des Schlauchs, auf den das gleiche Gitter mit den gleichen Abmessungen aufgezeichnet wurde, nachempfunden, in dem der Schlauch mit der Vorrichtung um einen bestimmten Torsionswinkel tordiert wurde. Es zeigte sich eine optimale Übereinstimmung der Gitterlinien auf dem Unterarm des Patienten und dem Schlauch bei einem Torsionswinkel von 120°.

Darüber hinaus weist die Vorrichtung eine Messeinheit 23 zum Messen der elektrischen Eigenschaften des Feuchtigkeitssensors 80 auf, der mittig auf den Schlauch 20 aufgebracht wird. Die Messergebnisse werden in einer Auswerteinheit 24 der Vorrichtung nach den bekannten Verfahren ausgewertet.

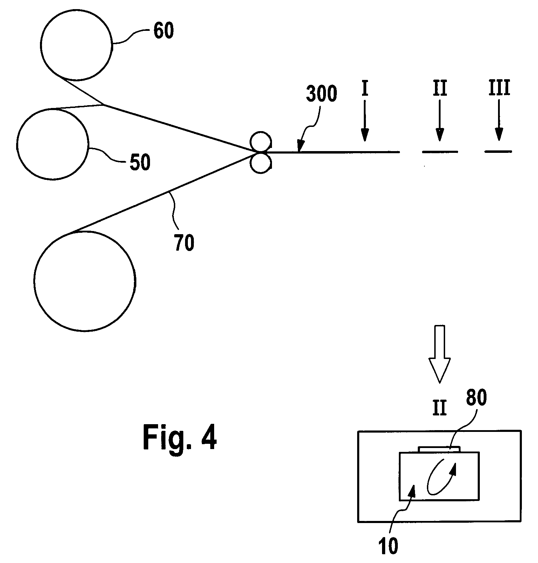

Fig. 4 zeigt die wesentlichen Verfahrensschritte zur Herstellung von zu überprüfenden Feuchtigkeitssensoren in stark vereinfachter schematischer Darstellung nach dem erfindungsgemäßen Produktionsverfahrens. Zur Herstellung einer Gewebebahn 300 mit einer Vielzahl von Feuchtigkeitssensoren, die jeweils eine gewebte elektrisch leitende Struktur aufweisen, werden die Kettfäden 50 und die Schussfäden 60 zugeführt. Nach der Herstellung des Gewebes aus leitfähigen und nicht-leitfähigen Kett- und Schussfäden erfolgen weitere dem Fachmann bekannte Verfahrensschritte. Dazu zählt beispielsweise das Veredeln, insbesondere das Waschen, das Fixieren oder eine Wärmebehandlung. Während des Webprozesses wird eine Lage 70 zugeführt, mit der die Gewebebahn 300 kaschiert wird. Die Lage 70 wird auf die Unterseite der Gewebebahn 300 aufgebracht. Dann werden in einem weiteren Verfahrensschritt I die einzelnen Feuchtigkeitssensoren separiert. In einem weiteren Prozessschritt II werden die Feuchtigkeitssensoren überprüft. Schließlich werden die Sensoren konfektioniert III.

Das oben beschriebene Verfahren zur Herstellung von gewebten Feuchtigkeitssensoren gehört mit Ausnahme der erfindungsgemäßen Prüfung der Feuchtigkeitssensoren zum Stand der Technik. Dieses Verfahren ist im Einzelnen in der WO 2011/116943 beschrieben.

Nachfolgend wird die Überprüfung der Feuchtigkeltssensoren mit dem erfindungsgemäßen Prüfverfahren beschrieben. Das erfindungsgemäße Prüfverfahren sieht eine Endkontrolle (EK) während der laufenden Produktion in dem Prozessschritt II vor. Einer Charge von produzierten Feuchtigkeitssensoren werden für eine Stichprobe ein oder mehrere Sensoren 80 entnommen, um die Sensoren mit der erfindungsgemäßen Vorrichtung 10 zu prüfen. Der zu prüfende Feuchtigkeitssensor 80 wird auf die Mantelfläche des Schlauchs 20 mittig aufgeklebt. Anschließend wird der Schlauch in die Betätigungseinheit 15 eingesetzt, wobei die Schlauchenden auf die zylindrischen Körper 12, 13 aufgeschoben und mittels der Befestigungsmittel 21, 22 befestigt werden. Daraufhin wird der Prüfzyklus gestartet, indem der Schlauch und der Feuchtigkeitssensor einer vorgegebenen Anzahl von Torsionen, vorzugsweise 400 bis 600 Torsionen, um einen vorgegebenen Torsionswinkel, vorzugsweise etwa 120°, unterworfen wird.

Die elektrischen Eigenschaften des Feuchtigkeitssensors 80 werden mit der Messeinheit 23 gemessen und mit der Auswerteinheit 24 ausgewertet, in dem die gemessenen Eigenschaften mit als Referenzwert vorgegebenen Eigenschaften verglichen werden. Bei einer Abweichung um ein vorgegebenes Maß, wird auf die Fehlerhaftigkeit des Feuchtigkeitssensors geschlossen. Die Auswerteinheit 24 verfügt vorzugsweise über eine Anzeigeeinheit, auf der das Ergebnis der Prüfung angezeigt oder protokolliert wird. Die gemessene Eigenschaft ist bei dem vorliegenden Ausführungsbeispiel zur Prüfung der gewebten Feuchtigkeitssensoren, der elektrische Widerstand zwischen den betreffenden Anschlüssen oder eine mit dem Widerstand korrelierende Größe. Folglich wird der elektrische Widerstand gemessen. Die gemessenen Widerstandswerte können statistisch ausgewertet werden. Beispielsweise können die Widerstandswerte mit einem oberen und unteren Grenzwert verglichen werden, wobei auf einen fehlerhaften Feuchtigkeitssensor geschlossen wird, wenn der gemessene Widerstand außerhalb des Grenzwertintervalls liegt.

Die erfindungsgemäße Vorrichtung erlaubt eine Erfassung der Messwerte vor und/oder während und/oder nach einer oder mehreren Torsionen: Mit Messungen vor den Torsionen lassen sich auch entsprechende Vergleichswerte für eine vorzugsweise statistische Auswertung ermitteln.

## Patentansprüche

1. Verfahren zum Prüfen von auf die Haut eines Patienten aufzubringenden Sensoren zum Erkennen von Flüssigkeit oder Feuchtigkeit, die eine elektrisch leitende Struktur mit elektrischen Anschlüssen aufweisen, wobei zur Prüfung der Feuchtigkeitssensoren dessen elektrische Eigenschaften unter definierten Bedingungen gemessen und mit als Referenzwert vorgegebenen elektrischen Eigenschaften verglichen werden,
**dadurch gekennzeichnet, dass**
aus der Produktion mindestens ein Feuchtigkeitssensor (80) entnommen wird, dass der mindestens eine Feuchtigkeitssensor auf die Oberfläche eines elastischen Körpers (20) aufgebracht und der elastische Körper mehrfach verformt wird, und nach dem mehrfachen Verformen des elastischen Körpers die gemessenen elektrischen Eigenschaften mit den als Referenzwert vorgegebenen elektrischen Eigenschaften verglichen werden, wobei auf eine Fehlerhaftigkeit der Feuchtigkeitssensoren auf der Grundlage der Abweichungen zwischen den gemessenen elektrischen Eigenschaften und den als Referenzwert vorgegebenen elektrischen Eigenschaften des mindestens einen Feuchtigkeitssensors geschlossen wird.

2. Verfahren nach Anspruch 1, **dadurch gekennzeichnet, dass** der mindestens eine Feuchtigkeitssensor auf die Oberfläche eines elastischen Torsionskörpers (20) aufgebracht und der Torsionskörper mehrfach tordiert wird.

3. Verfahren nach Anspruch 1, **dadurch gekennzeichnet, dass** der Torsionskörper (20) an einem Ende fest eingespannt wird und an dem anderen Ende um einen vorgegebenen Drehwinkel verdreht wird.

4. Verfahren nach Anspruch 1 oder 2, **dadurch gekennzeichnet, dass** der Torsionskörper ein zylindrischer Körper (20) ist, auf dessen Mantelfläche der Feuchtigkeitssensor aufgebracht wird.

5. Verfahren nach einem der Ansprüche 2 bis 4, **dadurch gekennzeichnet, dass** der Torsionskörper ein Schlauch (20) ist.

6. Verfahren nach einem der Ansprüche 1 bis 5, **dadurch gekennzeichnet, dass** eine Mehrzahl von Feuchtigkeitssensoren einer Charge entnommen werden und die Abweichungen zwischen den gemessenen elektrischen Eigenschaften und den als Referenzwert vorgegebenen elektrischen Eigenschaften der einer Charge entnommenen Feuchtigkeitssensoren statistisch ausgewertet werden.

7. Verfahren nach einem der Ansprüche 2 bis 6, **dadurch gekennzeichnet, dass** der Torsionskörper (20) um einen Torsionswinkel von 60° bis 180°, insbesondere 80° bis 160°, vorzugsweise 100° bis 140° tordiert wird.

8. Verfahren nach einem der Ansprüche 2 bis 7, **dadurch gekennzeichnet, dass** die Anzahl der Torsionen zwischen 100 und 1000, insbesondere 200 bis 800, vorzugsweise 400 bis 600 Torsionen ist.

9. Verfahren zum Produzieren von auf die Haut eines Patienten aufzubringenden Sensoren zum Erkennen von Feuchtigkeit, die eine elektrisch leitende Struktur mit elektrischen Anschlüssen aufweisen,
Herstellen einer Vielzahl von auf einer gemeinsamen Bahn nebeneinander angeordneten Feuchtigkeitssensoren,
Vereinzeln der Feuchtigkeitssensoren,
und Durchführen einer Prüfung der Feuchtigkeitssensoren nach einem Verfahren nach einem der Ansprüche 1 bis 8.

10. Verfahren nach Anspruch 9, **dadurch gekennzeichnet, dass** die Feuchtigkeitssensoren resistive Feuchtigkeitssensoren sind, wobei der elektrische Widerstand oder eine mit dem elektrischen Widerstand korrelierende Größe gemessen wird.

11. Verfahren nach Anspruch 9 oder 10, **dadurch gekennzeichnet, dass** der Feuchtigkeitssensor als ein textiles Flächengebilde aus nicht-leitfähigen Kettfäden und nicht-leitfähigen Schussfäden sowie leitfähigen Kettfäden und leitfähigen Schussfäden ausgebildet ist, wobei die nicht-leitfähigen Kettfäden und Schussfäden sowie leitfähigen Kettfäden und Schussfäden derart angeordnet sind, dass die elektrisch leitende Struktur ausgebildet ist.

12. Verfahren nach einem der Ansprüche 9 oder 10, **dadurch gekennzeichnet, dass** der Feuchtigkeitssensor ein Trägermaterial aufweist, auf das die elektrisch leitende Struktur aufgebracht, vorzugsweise aufgedruckt, ist.

13. Vorrichtung zur Durchführung einer Prüfung der Feuchtigkeitssensoren nach einem Verfahren nach einem der Ansprüche 1 bis 12, **dadurch gekennzeichnet, dass** die Vorrichtung zur Durchführung der Prüfung aufweist:
einen elastischen Torsionskörper (20) zum Aufbringen des Feuchtigkeitssensors,
eine Betätigungseinheit (15) zur auswechselbaren Aufnahme des Torsionskörpers (20), die derart ausgebildet ist, dass der Torsionskörper tordiert wird,
eine Steuereinheit (18) zum Ansteuern der Betätigungseinheit (15),
eine Messeinheit (23) zum Messen der elektrischen Eigenschaften des Feuchtigkeitssensors, und
eine Auswerteinheit (24) zum Auswerten der Messergebnisse der Messeinheit (12).

14. Vorrichtung nach Anspruch 13, **dadurch gekennzeichnet, dass** der Torsionskörper ein Schlauch (20) ist.

15. Vorrichtung nach Anspruch 14, **dadurch gekennzeichnet, dass** die Betätigungseinheit (25) einen drehbaren zylindrischen Körper (12) aufweist, an dem das eine Ende des Schlauchs (20) befestigbar ist, und einen feststehenden zylindrischen Körper (13) aufweist, an dem das andere Ende des Schlauchs befestigbar ist.

16. Vorrichtung nach einem der Ansprüche 13 bis 15, **dadurch gekennzeichnet, dass** die Steuereinheit (18) derart ausgebildet ist, dass die Betätigungseinheit (15) derart angesteuert wird, dass die Betätigungseinheit eine vorgegebene Anzahl von Torsionen des Torsionskörpers vornimmt.

17. Vorrichtung nach einem der Ansprüche 13 bis 16, **dadurch gekennzeichnet, dass** die Steuereinheit (18) derart ausgebildet ist, dass die Betätigungseinheit (15) derart angesteuert wird, dass die Betätigungseinheit Torsionen um einen vorgegebenen Torsionswinkel vornimmt.

## Claims

1. Method for testing sensors which are to be applied to a patient's skin, are intended for detecting liquid or moisture and comprise an electrically conductive structure comprising electrical terminals, electrical properties of the moisture sensors being measured under defined conditions and compared with electrical properties, preset as a reference value, in order to test said moisture sensors,
**characterised in that**
at least one moisture sensor (80) is removed from production, **in that** the at least one moisture sensor is applied to the surface of a resilient body (20) and the resilient body is repeatedly deformed, and, once the resilient body has been repeatedly deformed, the measured electrical properties are compared with the electrical properties preset as a reference value, it being concluded that the moisture sensors are faulty on the basis of deviations between the measured electrical properties and the electrical properties, preset as a reference value, of the at least one moisture sensor.

2. Method according to claim 1, **characterised in that** the at least one moisture sensor is applied to the surface of a resilient torsion body (20) and the torsion body is repeatedly twisted.

3. Method according to claim 1, **characterised in that** the torsion body (20) is firmly clamped at one end and is twisted through a preset angle of rotation at the other end.

4. Method according to either claim 1 or claim 2, **characterised in that** the torsion body is a cylindrical body (20), on the lateral surface of which the at least one moisture sensor is applied.

5. Method according to any of claims 2 to 4, **characterised in that** the torsion body is a hose (20).

6. Method according to any of claims 1 to 5, **characterised in that** a plurality of moisture sensors is removed from a batch, and the deviations between the measured electrical properties and the electrical properties, preset as a reference value, of the moisture sensors removed from the batch are evaluated statistically.

7. Method according to any of claims 2 to 6, **characterised in that** the torsion body (20) is twisted through an angle of twist of from 60° to 180°, in particular 80° to 160°, preferably 100° to 140°.

8. Method according to any of claims 2 to 7, **characterised in that** the number of twists is between 100 and 1000, in particular 200 to 800, preferably 400 to 600.

9. Method for producing sensors which are to be applied on a patient's skin, are intended for detecting moisture and comprise an electrically conductive structure comprising electrical terminals,
producing a plurality of sensors which are arranged one next to the other on a common web,
separating the moisture sensors, and
testing the moisture sensors according to a method according to any of claims 1 to 8.

10. Method according to claim 9, **characterised in that** the moisture sensors are resistive moisture sensors, the electrical resistance or a quantity correlating with the electrical resistance being measured.

11. Method according to either claim 9 or claim 10, **characterised in that** the moisture sensor is designed as a textile fabric consisting of non-conductive warp threads and nonconductive weft threads and conductive warp threads and conductive weft threads, the non-conductive warp threads and weft threads and the conductive warp threads and weft threads being arranged such that the electrically conductive structure is formed.

12. Method according to either claim 9 or claim 10, **characterised in that** the moisture sensor comprises a carrier material, onto which the electrically conductive structure is applied, preferably printed.

13. Device for testing moisture sensors according to a method according to any of claims 1 to 12, **characterised in that** the testing device comprises:
a resilient torsion body (20) for being applied to the moisture sensor,
an actuation unit (15) for interchangeably receiving the torsion body (20), designed such that the torsion body is twisted,
a control unit (18) for controlling the actuation unit (15),
a measuring unit (23) for measuring the electrical properties of the moisture sensor, and
an evaluation unit (24) for evaluating the measurement results from the measuring unit (12).

14. Device according to claim 13, **characterised in that** the torsion body is a hose (20).

15. Device according to claim 14, **characterised in that** the actuation unit (25) comprises a rotatable cylindrical body (12) to which one end of the hose (20) can be fastened, and a stationary cylindrical body (13) to which the other end of the hose can be fastened.

16. Device according to any of claims 13 to 15, **characterised in that** the control unit (18) is designed such that the actuation unit (15) is controlled such that the actuation unit performs a preset number of twists on the torsion body.

17. Device according to any of claims 13 to 16, **characterised in that** the control unit (18) is designed such that the actuation unit (15) is controlled such that the actuation unit performs twists through a preset angle of twist.

## Revendications

1. Procédé pour le contrôle de capteurs à appliquer sur la peau d'un patient, pour la détection d'un liquide ou d'humidité, qui présentent une structure électro-conductrice avec des branchements électriques, pour le contrôle des capteurs d'humidité, leurs propriétés électriques étant mesurées dans des conditions données et comparées à des propriétés électriques prédéterminées sous la forme d'une valeur de référence,
**caractérisé en ce que**
au moins un capteur d'humidité (80) est prélevé dans la production, **en ce que** l'au moins un capteur d'humidité est appliqué sur la surface d'un corps élastique (20) et le corps élastique est déformé plusieurs fois et, après les multiples déformations du corps élastique, les propriétés électriques mesurées sont comparées avec les propriétés électriques prédéterminées sous la forme d'une valeur de référence, les écarts entre les propriétés électriques mesurées et les propriétés électriques prédéterminées sous la forme d'une valeur de référence, de l'au moins un capteur d'humidité permettant de déduire une défectuosité des capteurs d'humidité.

2. Procédé selon la revendication 1, **caractérisé en ce que** l'au moins un capteur d'humidité est appliqué sur la surface d'un corps de torsion élastique (20) et le corps de torsion est tordu plusieurs fois.

3. Procédé selon la revendication 1, **caractérisé en ce que** le corps de torsion (20) est serré fermement à une extrémité et, à l'autre extrémité, il est soumis à une rotation d'un angle prédéterminé.

4. Procédé selon la revendication 1 ou 2, **caractérisé en ce que** le corps de torsion est un corps cylindrique (20) sur la surface extérieure duquel le capteur d'humidité est appliqué.

5. Procédé selon l'une des revendications 2 à 4, **caractérisé en ce que** le corps de torsion est un tuyau (20).

6. Procédé selon l'une des revendications 1 à 5, **caractérisé en ce qu'**une pluralité de capteurs d'humidité d'un lot sont prélevés et les écarts entre les propriétés électriques mesurées et les propriétés électriques prédéterminées sous la forme d'une valeur de référence des capteurs d'humidité prélevés dans un lot sont analysés statistiquement.

7. Procédé selon l'une des revendications 2 à 6, **caractérisé en ce que** le corps de torsion (20) est tordu d'un angle de torsion de 60° à 180°, plus particulièrement de 80° à 160°, de préférence de 100° à 140°.

8. Procédé selon l'une des revendications 2 à 7, **caractérisé en ce que** le nombre de torsion est entre 100 et 1000, plus particulièrement de 200 à 800, de préférence de 400 à 600 torsions.

9. Procédé de production de capteurs à appliquer sur la peau d'un patient pour la détection d'humidité, qui présentent une structure électro-conductrice avec des branchements électriques,
fabrication d'un grand nombre de capteurs d'humidité juxtaposés sur une bande commune, séparation des capteurs d'humidité,
et réalisation d'un contrôle des capteurs d'humidité selon un procédé selon l'une des revendications 1 à 8.

10. Procédé selon la revendication 9, **caractérisé en ce que** les capteurs d'humidité sont des capteurs d'humidité résistifs, la résistance électrique ou une grandeur corrélée avec la résistance électrique étant mesurée.

11. Procédé selon la revendication 9 ou 10, **caractérisé en ce que** le capteur d'humidité est conçu comme une structure plane textile constituée de fils de chaîne non conducteurs et de fils de trame non conducteurs ainsi que de fils de chaîne conducteurs et de fils de trame conducteurs, les fils de chaîne et de trame non conducteurs et les fils de chaîne et de trame conducteurs étant disposés de façon à ce que la structure électro-conductrice soit obtenue.

12. Procédé selon l'une des revendications 9 ou 10, **caractérisé en ce que** le capteur d'humidité comprend un matériau de support sur lequel la structure électro-conductrice est appliquée, de préférence imprimée.

13. Dispositif pour la réalisation d'un contrôle des capteurs d'humidité selon un procédé selon l'une des revendications 1 à 12, **caractérisé en ce que** le dispositif de réalisation du contrôle comprend :
un corps de torsion élastique (20) pour l'application du capteur d'humidité,
une unité d'actionnement (15) pour le logement interchangeable du corps de torsion (20),
qui est conçue de façon à ce que le corps de torsion soit tordu,
une unité de commande (18) pour le pilotage de l'unité d'actionnement (15),
une unité de mesure (23) pour la mesure des propriétés électriques du capteur d'humidité, et
une unité d'analyse (24) pour l'analyse des résultats de mesure de l'unité de mesure (12).

14. Dispositif selon la revendication 13, **caractérisé en ce que** le corps de torsion est un tuyau (20).

15. Dispositif selon la revendication 14, **caractérisé en ce que** l'unité d'actionnement (25) comprend un corps cylindrique rotatif (12) auquel une extrémité du tuyau (20) peut être fixée, et un corps cylindrique fixe (13) auquel l'autre extrémité du tuyau peut être fixée.

16. Dispositif selon l'une des revendications 13 à 15, **caractérisé en ce que** l'unité de commande (18) est conçue de façon à ce que l'unité d'actionnement (15) soit pilotée de façon à ce que l'unité d'actionnement effectue un nombre prédéterminé de torsions du corps de torsion.

17. Dispositif selon l'une des revendications 13 à 16, **caractérisé en ce que** l'unité de commande (18) est conçue de façon à ce que l'unité d'actionnement (15) soit pilotée de façon à ce que l'unité d'actionnement effectue des torsions d'un angle de torsion prédéterminé.
